(19) Europäisches Patentamt
European Patent Office
Office européen des brevets

(11) **EP 1 965 914 B1**

(12) **FASCICULE DE BREVET EUROPEEN**

(45) Date de publication et mention
de la délivrance du brevet:
**17.06.2009 Bulletin 2009/25**

(51) Int Cl.:
***B01J 29/80*** *(2006.01)* ***C07C 5/27*** *(2006.01)*

(21) Numéro de dépôt: **06841855.7**

(86) Numéro de dépôt international:
**PCT/FR2006/002647**

(22) Date de dépôt: **01.12.2006**

(87) Numéro de publication internationale:
**WO 2007/080246 (19.07.2007 Gazette 2007/29)**

(54) **CATALYSEUR COMPRENANT UNE ZEOLITHE EUO, UNE ZEOLITHE 10 MR ET UNE ZEOLITHE 12 MR ET SON UTILISATION EN ISOMERISATION DES COMPOSES C8 AROMATIQUES**

KATALYSATOR, UMFASSEND EINEN EUO-ZEOLITH, EINEN 10-MR-ZEOLITH UND EINEN 12-MR-ZEOLITH, UND SEINE VERWENDUNG BEI DER ISOMERISIERUNG VON AROMATISCHEN C8-VERBINDUNGEN

CATALYST COMPRISING AN EUO ZEOLITE, A 10 MR ZEOLITE AND A 12 MR ZEOLITE AND ITS USE IN THE ISOMERISATION OF AROMATIC C8 COMPOUNDS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **22.12.2005 FR 0513176**

(43) Date de publication de la demande:
**10.09.2008 Bulletin 2008/37**

(73) Titulaire: **Institut Français du Pétrole**
**92852 Rueil Malmaison Cedex (FR)**

(72) Inventeurs:
• **GUILLON, Emmanuelle**
**F-69390 Vernaison (FR)**
• **SANCHEZ, Eric**
**F-69230 Saint Genis Laval (FR)**
• **LACOMBE, Sylvie**
**F-69390 Vernaison (FR)**

(56) Documents cités:
**EP-A- 0 923 987** **WO-A-00/66263**
**WO-A-99/28031** **US-A- 4 537 754**
**US-B1- 6 872 866**

• **MOREAU ET AL: "Ethylbenzene isomerization over bifunctional platinum alumina-EUO catalysts: Location of the active sites" MICROPOROUS AND MESOPOROUS MATERIALS, ELSEVIER SCIENCE PUBLISHING, NEW YORK, US, vol. 90, no. 1-3, 18 octobre 2005 (2005-10-18), pages 327-338, XP005297671 ISSN: 1387-1811**

**Description**

Domaine technique

La présente invention se rapporte à un catalyseur formé d'au moins trois zéolithes distinctes et utilisable par exemple dans les réactions de transformation des hydrocarbures aromatiques. De manière plus précise, elle concerne un catalyseur d'isomérisation des composés C8 aromatiques. La présente invention concerne aussi l'utilisation dudit catalyseur dans un procédé d'isomérisation d'une charge comprenant des composés aromatiques à 8 atomes de carbone par molécule.

Art antérieur

Selon les procédés connus d'isomérisation des composés aromatiques à huit atomes de carbone (AC8), une charge généralement pauvre en paraxylène par rapport à l'équilibre thermodynamique du mélange (c'est-à-dire dont la teneur en paraxylène est nettement inférieure à celle du mélange à l'équilibre thermodynamique à la température considérée, ce mélange comprenant au moins un composé choisi dans le groupe formé par le métaxylène, l'orthoxylène, le paraxylène et l'éthylbenzène) et généralement riche en éthylbenzène par rapport à ce même mélange à l'équilibre thermodynamique est introduite dans un réacteur contenant au moins un catalyseur, dans des conditions de température et de pression adéquates pour obtenir une composition, à la sortie dudit réacteur, en composés aromatiques à huit atomes de carbone la plus proche possible de la composition dudit mélange à l'équilibre thermodynamique à la température du réacteur. Pour obtenir une telle composition, l'Homme du métier s'efforce généralement de maximiser la conversion en éthylbenzène présent dans la charge. A partir du mélange obtenu en sortie du réacteur d'isomérisation, on sépare le xylène et éventuellement le métaxylène ou l'orthoxylène qui sont les isomères recherchés car ils présentent un intérêt important notamment pour l'industrie des fibres synthétiques.

Les catalyseurs utilisés pour la mise en oeuvre d'un procédé d'isomérisation de composés aromatiques à huit atomes de carbone sont généralement des catalyseurs zéolithiques. Les catalyseurs de l'état de la technique, en particulier les catalyseurs à base de zéolithe mordénite, ne donnent que des performances catalytiques médiocres car ils conduisent à des réactions parasites non négligeables générant des pertes. Par exemple, on peut citer, parmi ces réactions secondaires, l'ouverture de cycles naphténiques suivie ou non de craquage (pertes vers les paraffines) ou encore les réactions de dismutation et de transalkylation des aromatiques à huit atomes de carbone (pertes vers des composés aromatiques non recherchés) ou bien encore l'hydrogénation des composés aromatiques (pertes vers les naphtènes). Des catalyseurs à base de zéolithe ZSM-5, seule ou en mélange avec d'autres zéolithes comme la mordénite par exemple, ont déjà été utilisés mais ne donnent pas, non plus, des performances catalytiques optimales. Plus récemment, il a été proposé un catalyseur à base d'une zéolithe de type structural EUO (EP-A1-0.923.987).

Aussi, la présente invention se propose de fournir un nouveau catalyseur présentant une composition telle que lorsqu'il est utilisé pour l'isomérisation des composés aromatiques à 8 atomes de carbone par molécule, la conversion en éthylbenzène est améliorée et les réactions secondaires sont limitées, réduisant en conséquence les pertes.

Résumé

La présente invention a pour objet un catalyseur comportant au moins une zéolithe de type structural EUO, au moins une zéolithe présentant des canaux dont l'ouverture est définie par un anneau à 10 atomes d'oxygène (10 MR), au moins une zéolithe présentant des canaux dont l'ouverture est définie par un anneau à 12 atomes d'oxygène (12 MR) et au moins une matrice minérale poreuse. De manière avantageuse, le catalyseur selon l'invention comporte au moins un métal choisi parmi les métaux des groupes VIB, VIIB et VIII de la classification périodique des éléments, et éventuellement en outre au moins un métal choisi parmi les métaux des groupes IIIA et IVA de la classification périodique des éléments. Chacune des zéolithes comprises dans le catalyseur selon l'invention contient du silicium et au moins un élément T choisi dans le groupe formé par l'aluminium, le fer, le gallium et le bore, de préférence l'aluminium.

La présente invention concerne également l'utilisation dudit catalyseur dans un procédé d'isomérisation d'une charge comprenant des composés aromatiques à 8 atomes de carbone par molécule.

Intérêt

Il a été découvert, de manière surprenante, qu'un catalyseur composite comprenant l'association d'au moins une zéolithe de type structural EUO, d'au moins une zéolithe présentant des canaux dont l'ouverture est définie par un anneau à 10 atomes d'oxygène (10 MR) et d'au moins une zéolithe présentant des canaux dont l'ouverture est définie par un anneau à 12 atomes d'oxygène (12 MR) conduit à des performances catalytiques améliorées dans les réactions d'isomérisation des composés aromatiques à 8 atomes de carbone par molécule. En particulier, le catalyseur selon l'invention conduit à une conversion en éthylbenzène supérieure à celle réalisée par les catalyseurs de l'état de la technique, notamment des catalyseurs à base d'une zéolithe de type structural EUO ou de type structural MOR. Par ailleurs, le catalyseur selon l'invention limite notablement les réactions secondaires, générant ainsi de moindres pertes, par rapport aux catalyseurs de l'état de la technique.

De plus, en ajustant la quantité relative des trois zéolithes, celle à type structural EUO, celle à 10 MR et celle à 12 MR, dans le catalyseur selon l'invention, il est possible de traiter une très large gamme de mélanges de charges hydrocarbonées.

Description

La présente invention a pour objet un catalyseur comportant au moins une zéolithe de type structural EUO, au moins une zéolithe présentant des canaux dont l'ouverture est définie par un anneau à 10 atomes d'oxygène (10 MR), au moins une zéolithe présentant des canaux dont l'ouverture est définie par un anneau à 12 atomes d'oxygène (12 MR) et au moins une matrice minérale poreuse.

Conformément à l'invention, le catalyseur comporte au moins trois zéolithes de type structural différent.

La zéolithe de type structural EUO, présente dans le catalyseur selon l'invention, est déjà décrite dans l'état de la technique. Elle présente un réseau microporeux monodimensionnel, dont le diamètre des pores est de 4,1 x 5,4 Å (1 Å = 1 Angström = $10^{-10}$ m) (« Atlas of Zeolite Structure Types », W.M. Meier, D.H. Olson, Ch. Baerlocher, 5ème Edition, 2001). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. La zéolithe de type structural EUO regroupe les zéolithes EU-1 (EP-B1-42 226), ZSM-50 (US 4.640.829) et TPZ-3 (EP-A1-51 318). La zéolithe de type structural EUO, présente dans le catalyseur selon l'invention, est de préférence une zéolithe EU-1.

Les zéolithes présentant des canaux dont l'ouverture est définie par un anneau à 10 atomes d'oxygène et celles présentant des canaux dont l'ouverture est définie par un anneau à 12 atomes d'oxygène sont définies dans la classification "Atlas of Zeolite Framework Types", W. M Meier, D. H. Olson et Ch. Baerlocher, 5th revised edition, 2001, Elsevier auquel se réfère également la présente demande. Les zéolithes y sont classées selon la taille de leurs ouvertures de pores ou canaux. Conformément à l'invention, au moins une zéolithe comprise dans le catalyseur selon l'invention présentent des pores ou canaux dont l'ouverture est définie par un anneau à 10 atomes d'oxygène (ouverture à 10MR) et au moins une autre zéolithe comprise dans le catalyseur selon l'invention présentent des pores ou canaux dont l'ouverture est définie par un anneau à 12 atomes d'oxygène (ouverture à 12MR). Selon l'invention, les canaux de la zéolithe ayant une ouverture à 10 MR appelée dans la suite de la description par zéolithe 10 MR sont des canaux principaux qui débouchent directement sur l'extérieur de ladite zéolithe. La zéolithe 10 MR peut également comporter des canaux secondaires internes, par exemple des canaux à 12 MR, accessibles uniquement par les canaux principaux à 10 MR. La zéolithe ayant une ouverture à 12 MR appelée dans la suite de la description par zéolithe à 12 MR présente au moins des canaux principaux 12 MR débouchant directement sur l'extérieur de ladite zéolithe.

La zéolithe de type structural EUO, les zéolithes 10 MR et 12 MR présentes dans le catalyseur selon l'invention comprennent du silicium et au moins un élément T choisi dans le groupe formé par l'aluminium, le fer, le gallium et le bore, de préférence l'aluminium. Elles sont, de préférence, pratiquement totalement, sous forme acide.

La zéolithe de type structural EUO, présente dans le catalyseur selon l'invention, est de préférence une zéolithe EU-1. Elle est caractérisée par un rapport atomique Si/T, de préférence un rapport atomique Si/Al, d'au moins 5, avantageusement compris entre 5 et 100. Ladite zéolithe de type structural EUO est au moins en partie, de préférence pratiquement totalement, sous forme acide, c'est-à-dire sous forme hydrogène H⁺, la teneur en sodium étant de préférence telle que le rapport atomique Na/T est inférieur à 0,1, de manière plus préférée inférieur à 0,05. Un mode de synthèse d'une zéolithe EU-1 est décrit dans le brevet EP-B1-42 226. Un mode de synthèse d'une zéolithe ZSM-50 est décrit dans le brevet US 4.640.829. Un mode de synthèse d'une zéolithe TPZ-3 est décrit dans la demande de brevet EP-A1-51 318.

La zéolithe 10 MR présente dans le catalyseur selon l'invention est caractérisée par un rapport atomique Si/T, de préférence un rapport atomique Si/Al, compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05 % poids par rapport au poids total de zéolithe sèche. Toutes les zéolithes possédant des canaux dont l'ouverture est définie par un anneau à 10 atomes d'oxygène (10 MR) et connues dans l'art antérieur conviennent pour la mise en oeuvre du catalyseur de la présente invention. La zéolithe 10 MR est avantageusement choisie parmi les zéolithes présentant le type structural MFI, TON, NES et FER. De manière très préférée, la zéolithe 10 MR est choisie parmi les zéolithes ZSM-5, NU-87, IM-5, ferrierite, NU-85 et ZSM-22. De manière très avantageuse, il s'agit de la zéolithe ZSM-5. Ces zéolithes et leur mode de préparation sont bien connus de l'Homme du métier ; la zéolithe NU-85 est notamment décrite dans le brevet US 5.446.234 ; la zéolithe IM-5 est particulièrement décrite dans les brevets EP 0.946.416 et US 6.136.290.

La zéolithe 12MR présente dans le catalyseur selon l'invention est caractérisée par un rapport atomique Si/T, de préférence un rapport atomique Si/Al, compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05% poids par rapport au poids total de zéolithe sèche.

Toutes les zéolithes possédant au moins des canaux (principaux ou secondaires) dont l'ouverture est définie par un anneau à 12 atomes d'oxygène (12 MR) et connues dans l'art antérieur conviennent pour la mise en oeuvre du catalyseur de la présente invention. La zéolithe 12 MR est avantageusement choisie parmi les zéolithes présentant le type structural BEA, MOR, MAZ, FAU, MTW et BOG. De manière très préférée, la zéolithe 12 MR est choisie parmi les zéolithes Béta, Y, mordénite, ZSM-12, la mazzite et la boggsite. La boggsite possède des canaux principaux à 10 MR et 12 MR. De manière très préférée, la zéolithe 12 MR, présente dans le catalyseur selon l'invention, est la mordénite ou la béta.

Le catalyseur selon l'invention comporte éventuellement au moins une zéolithe présentant des canaux dont l'ouverture est définie par un anneau à 8 atomes d'oxygène. Selon l'invention, les canaux de la zéolithe ayant une ouverture à 8 MR appelée dans la suite de la description par zéolithe 8 MR sont des canaux principaux qui débouchent directement sur l'extérieur de ladite zéolithe et qui ne possèdent pas de canaux à plus larges ouvertures de pores. Ladite zéolithe 8 MR présente avantageusement dans le catalyseur selon l'invention comprend du silicium et au moins un élément T choisi dans le groupe formé par l'aluminium, le fer, le gallium et le bore, de préférence l'aluminium. Elle se trouve, de préférence, pratiquement totalement, sous forme acide. Ladite zéolithe 8 MR est caractérisée par un rapport atomique Si/T, de préférence un rapport atomique Si/Al, compris entre 2 et 250, de préférence compris entre 5 et 150 et de manière très préférée compris entre 10 et 80. La teneur en sodium est inférieure à 0,2 % poids, de préférence inférieure à 0,1 % poids et de manière très préférée inférieure à 0,05 % poids par rapport au poids total de zéolithe sèche.

Toutes les zéolithes possédant des canaux dont l'ouverture est définie par un anneau à 8 atomes d'oxygène (8 MR) et connues dans l'art antérieur conviennent pour la mise en oeuvre du catalyseur de la présente invention. La zéolithe 8 MR est avantageusement choisie parmi les zéolithes présentant le type structural ERI, ESV et LTA. De manière préférée, les zéolithes 8 MR préférées sont choisies parmi les zéolithes erionite, ERS-7 et linde type A. Ces zéolithes et leur mode de préparation sont bien connus de l'Homme du métier.

Les cristaux de la zéolithe de type structural EUO, ceux de la, zéolithe 10MR, ceux de la zéolithe 12 MR et éventuellement ceux de la zéolithe 8MR, sont bien distincts les uns des autres : ils ne se recouvrent pas.

Les rapports atomiques Si/T, de préférence les rapports atomiques Si/Al, des zéolithes décrites ci-dessus sont ceux obtenus à l'issue de la synthèse desdites zéolithes ou bien obtenus après des traitements post-synthèse d'extraction d'une partie des atomes T, dits traitements de désalumination lorsque T est l'aluminium, bien connus de l'homme du métier, tels que et à titre non exhaustif les traitements hydrothermiques suivis ou non d'attaques acides ou bien encore les attaques acides directes par des solutions d'acides minéraux ou organiques visant à extraire une partie des atomes T, de préférence une partie des atomes d'aluminium, de la charpente zéolithique.

Le rapport atomique Si/T, de préférence le rapport atomique Si/Al, de la zéolithe de type structural EUO, des zéolithes 10 MR et 12 MR, et éventuellement 8 MR, entrant dans la composition du catalyseur selon l'invention ainsi que la composition chimique dudit catalyseur sont déterminés par fluorescence X et absorption atomique.

La zéolithe de type structural EUO, les zéolithes 10 MR et 12 MR, et éventuellement la zéolithe 8 MR, entrant dans la composition du catalyseur selon l'invention peuvent être calcinées et échangées par au moins un traitement par une solution d'au moins un sel d'ammonium de manière à obtenir la forme ammonium des zéolithes qui une fois calcinée conduisent à la forme hydrogène desdites zéolithes.

La zéolithe de type structural EUO, les zéolithes 10 MR et 12 MR et éventuellement la zéolithe 8 MR entrant dans la composition du catalyseur selon l'invention sont au moins en partie, de préférence pratiquement totalement, sous forme acide, c'est-à-dire sous forme hydrogène ($H^+$). Le rapport atomique Na/T est généralement inférieur à 10% et de préférence inférieur à 5% et de manière encore plus préférée inférieur à 1 %.

Les zéolithes 10 MR et 12MR, et éventuellement la zéolithe 8 MR, sont répertoriées dans l'atlas des Zéolithes et sont synthétisées selon les méthodes décrites dans les références citées dans cet ouvrage ("Atlas of Zeolite Framework Types", W. M Meier, D. H. Olson et

Ch. Baerlocher, 5th revised edition, 2001) ou par toute autre méthode décrite dans la littérature ouverte à l'Homme du métier. Toute zéolithe commerciale peut par ailleurs être utilisée pour obtenir le catalyseur de l'invention.

La matrice minérale poreuse présente dans le catalyseur selon l'invention est choisie généralement parmi les éléments du groupe formé par les argiles (par exemple parmi les argiles naturelles telles que le kaolin, la sepiolite, l'attapulgite ou la bentonite), la magnésie, les alumines, les silices, l'oxyde de titane, l'oxyde de bore, la zircone, les phosphates d'aluminium, les phosphates de titane, les phosphates de zirconium, les silice-alumines amorphes et le charbon, de préférence parmi les éléments du groupe formé par les alumines, les argiles, les mélanges d'alumine et de silice et les mélanges d'alumine et de silice-alumine, de manière encore plus préférée parmi les alumines et notamment l'alumine gamma.

De manière avantageuse, le catalyseur selon l'invention comporte au moins un métal choisi parmi les métaux des groupes VIB, VIIB et VIII de la classification périodique des éléments, de manière très avantageuse parmi les métaux des groupes VIIB et VIII et de manière encore plus avantageuse parmi les métaux du groupe VIII de la classification périodique des éléments. Préférentiellement, le métal du groupe VIB est le molybdène. Préférentiellement, le métal du groupe VIIB est le rhénium. Préférentiellement, le métal du groupe VIII est choisi parmi le platine et le palladium, de préférence il s'agit du platine. La teneur pondérale dudit élément est de préférence comprise entre 0,01 et 5 % poids, de manière préférée entre 0,01 et 2 % poids et de manière encore plus préférée entre 0,05 et 1 % poids par rapport au poids total dudit catalyseur.

Selon l'invention, la composition catalytique du catalyseur contient avantageusement au moins un métal du groupe VIII de la classification périodique des éléments, de préférence choisi dans le groupe formé par le platine et le palladium, de manière encore plus préférée ledit métal du groupe VIII est le platine. La teneur pondérale dudit métal du groupe VIII est généralement comprise entre 0,01 et 2,0% poids, de préférence entre 0,05 et 1,0% poids par rapport au poids total du catalyseur. De manière préférée, la dispersion du métal du groupe VIII, de préférence le platine, déterminée par chimisorption, par exemple par titration $H_2$-$O_2$ ou par chimisorption du monoxyde de carbone, sur le catalyseur est supérieure à 50%, de manière très préférée supérieure à 70%.

De manière très avantageuse, ladite composition catalytique du catalyseur selon l'invention contient, en plus d'au moins un métal choisi parmi les métaux des groupes VIB, VIIB et VIII, au moins un métal choisi parmi les métaux des groupes IIIA et IVA. Ledit métal choisi parmi les métaux des groupes IIIA et IVA est présent en une teneur comprise entre 0,01 et 5,0% poids, de préférence comprise entre 0,05 et 1,0 % poids par rapport au poids total du catalyseur. Préférentiellement, le métal du groupe IIIA est l'indium. Préférentiellement, le métal du groupe IVA est l'étain.

Le catalyseur selon l'invention, de préférence mis sous forme de billes ou d'extrudés et plus préférentiellement sous forme d'extrudés, contient par rapport au poids du catalyseur : - de 1 à 90%, de préférence de 3 à 60% et de manière très préférée de 3 à 40 % d'au moins une zéolithe de type structural EUO, d'au moins une zéolithe présentant des canaux dont l'ouverture est définie par un anneau à 10 atomes d'oxygène (10 MR) et d'au moins une zéolithe présentant des canaux dont l'ouverture est définie par un anneau à 12 atomes d'oxygène (12 MR), éventuellement d'au moins une zéolithe présentant des canaux dont l'ouverture est définie par un anneau à 8 atomes d'oxygène (8 MR),

- de 0 à 5,0%, de préférence de 0,01 à 5,0 %, de manière très préférée de 0,01 à 2,0 % et de manière encore plus préférée de 0,05 à 1,0 % d'au moins un métal hydro-deshydrogénant choisi parmi les métaux des groupes VIB,VIIB et VIII.
- éventuellement au moins un métal additionnel choisi parmi les métaux des groupes IIIA et IVA dont la teneur pondérale est comprise entre 0,01 et 5,0 %, de préférence entre 0,05 et 1,0%,
- éventuellement du soufre,
- au moins une matrice minérale poreuse, dite liant, assurant le complément à 100% dans le catalyseur. La matrice minérale poreuse, est choisie généralement parmi les éléments du groupe formé par les argiles (par exemple parmi les argiles naturelles telles que le kaolin, la sepiolite, l'attapulgite ou la bentonite), la magnésie, les alumines, les silices, l'oxyde de titane, l'oxyde de bore, la zircone, les phosphates d'aluminium, les phosphates de titane, les phosphates de zirconium, les silice-alumines amorphes et le charbon, de préférence parmi les éléments du groupe formé par les alumines, les argiles, les mélanges d'alumine et de silice et les mélanges d'alumine et de silice-alumine, de manière encore plus préférée parmi les alumines et notamment l'alumine gamma.

De manière générale et selon un premier mode de réalisation du procédé de préparation du catalyseur selon l'invention, le catalyseur est préparé par mélange d'au moins une zéolithe de type structural EUO, d'au moins une zéolithe 10 MR et d'au moins une zéolithe 12 MR, éventuellement d'au moins une zéolithe 8 MR, lesdites zéolithes se trouvant à l'état de poudre. Le mélange desdites zéolithes est réalisé par toutes les techniques de mélange de poudres connues de l'Homme du métier. Une fois le mélange des poudres de zéolithes, réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ses formes connues de l'Homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées. Après l'étape de mise en forme, le catalyseur obtenu est soumis à une étape de séchage réalisée à une température comprise entre 80 et 150°C puis à une étape de calcination réalisée à une température comprise entre 300 et 600°C, de préférence entre 400 et 550°C.

Dans le cas où le catalyseur selon l'invention comporte au moins un métal choisi parmi les métaux des groupes VIB,VIIB et VIII de la classification périodique des éléments, le(s)dit(s) métal(aux) est(sont) déposé(s) sur un support catalytique après la mise en forme des zéolithes exemptes de métaux, par tout procédé connu de l'homme du métier et permettant le dépôt de métal sur le support catalytique. On désigne par "support catalytique", le mélange de zéolithes (exemptes de métaux) avec au moins une matrice minérale poreuse après mise en forme, séchage et calcination, comme décrit ci-dessus. Ledit support catalytique du catalyseur de la présente invention renferme généralement les teneurs suivantes en matrice et zéolithes :

- 1 à 90% poids, de préférence de 3 à 60% poids, de manière plus préférée de 3 à 40% poids de zéolithes telles que au moins une zéolithe soit une zéolithe de type structural EUO, au moins une zéolithe soit choisie parmi les zéolithes 10 MR, au moins une zéolithe soit choisie parmi les zéolithes 12 MR et éventuellement au moins une zéolithe soit choisie parmi les zéolithes 8 MR,
- 10 à 99% poids, de préférence de 40 à 97% poids, de manière plus préférée de 60 à 97% poids d'au moins une matrice minérale poreuse amorphe ou mal cristallisée de type oxyde.

Un deuxième mode de réalisation du procédé de préparation du catalyseur selon l'invention, dans le cas préféré où ledit catalyseur comporte au moins un métal choisi parmi les métaux des groupes VIB, VIIB et VIII de la classification périodique des éléments consiste à soumettre au moins une des zéolithes précédemment décrites et comprises dans ledit catalyseur au dépôt d'au moins un métal choisi parmi les métaux des groupes VIB, VIIB et VIII, préalablement à la mise en forme de l'ensemble des zéolithes.

De préférence, au moins la zéolithe EU-1 est soumise au dépôt d'au moins un métal choisi parmi les métaux des groupes VIB, VIIB et VIII. Il est également avantageux de soumettre la zéolithe 10 MR au dépôt d'au moins un métal choisi parmi les métaux des groupes VIB, VIIB et VIII et de soumettre la zéolithe 12 MR au dépôt d'au moins un autre métal choisi parmi les métaux des groupes VIB, VIIB et VIII. Il est encore avantageux, conformément au deuxième mode de réalisation du procédé de préparation du catalyseur selon l'invention, de soumettre chacune des zéolithes présentes dans le catalyseur selon l'invention au dépôt d'au moins un même métal choisi parmi les métaux des groupes VIB, VIIB et VIII, de préférence au dépôt d'au moins un métal du groupe VIII et de manière plus préférée au dépôt de platine. Le mélange de ces zéolithes qui sont alors à l'état de poudre, au moins une des zéolithes étant chargée en métal(ux), est réalisé par toutes les techniques de mélange de poudres connues de l'homme du métier. Une fois le mélange des poudres de zéolithes, dont l'une au moins est chargée en métal(ux), réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ces formes connues de l'Homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées. Après l'étape de mise en forme, le produit obtenu est soumis à une étape de séchage à une température comprise entre 80 et 150°C puis à une étape de calcination à une température comprise entre 300 et 600°C, de préférence entre 400 et 550°C.

Pour le dépôt de métal sur au moins une zéolithe telle que décrite précédemment et / ou sur le support catalytique selon le premier ou le deuxième mode de réalisation du procédé de préparation du catalyseur selon l'invention décrit ci-dessus, on peut utiliser la technique d'échange cationique avec compétition où le compétiteur est de préférence le nitrate d'ammonium, le rapport de compétition entre le compétiteur et le précurseur métallique étant au moins égal à environ 5 et avantageusement d'environ 5 à 200. On peut utiliser aussi la technique d'imprégnation à sec ou de coprécipitation.

Les sources des métaux du groupe VIII qui peuvent être utilisées sont bien connues de l'Homme du métier. Par exemple, on utilisera les nitrates, les sulfates, les phosphates, les halogénures par exemple chlorures, bromures et fluorures, les carboxylates par exemple acétates et carbonates. Dans le cas du platine, on utilise préférentiellement l'acide hexachloroplatinique ou le chlorure de platine tétramine. Les sources des métaux du groupe VIIB qui peuvent être utilisées sont également bien connues de l'Homme du métier. Dans le cas du rhénium, on utilise habituellement un complexe de perrhénate d'ammonium $(NH_4)ReO_4$ ou l'acide perrhénique. Les sources des métaux du groupe VIB qui peuvent être utilisées sont également bien connues de l'Homme du métier. Dans le cas du molybdène, on peut utiliser les acides molybdiques et leurs sels en particulier lés sels d'ammonium tels que le molybdate d'ammonium, l'heptamolybdate d'ammonium ainsi que l'acide phosphomolybdique. De préférence, on utilise l'heptamolybdate d'ammonium $(NH_4)_6Mo_7O_{24}$. Le dépôt du ou des métal(ux) est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures, de préférence on opérera entre 350° et 550°C pendant 2 à 5 heures.

Selon le premier mode ou le deuxième mode de réalisation de préparation du catalyseur selon l'invention, et dans le cas préféré où ledit catalyseur comporte au moins un métal choisi parmi les métaux des groupes VIB, VIIB et VIII de la classification périodique des éléments et éventuellement au moins un métal additionnel choisi parmi les métaux des groupes IIIA et IVA, on peut déposer le métal ou les métaux non plus directement sur une ou plusieurs zéolithes, mais sur la matrice minérale poreuse (par exemple le liant aluminique) du support catalytique formé des trois zéolithes, éventuellement des quatre zéolithes, et d'au moins une matrice, avant ou après l'étape de mise en forme, en mettant en oeuvre un échange anionique. On peut citer par exemple dans le cas du dépôt de platine l'utilisation du complexe hexachloroplatinique $H_2PtCl_6$ et dans le cas du dépôt de rhénium l'utilisation de l'acide perrhénique $HReO_4$. En général après le dépôt de métal(ux), le catalyseur est comme précédemment soumis à une calcination puis réduit sous hydrogène comme indiqué ci-dessus.

Dans le cas où le catalyseur selon l'invention contient plusieurs métaux, ces derniers peuvent être introduits soit tous de la même façon soit par des techniques différentes, avant ou après mise en forme selon le mode de réalisation du procédé de préparation du catalyseur employé et dans n'importe quel ordre. Dans le cas où la technique utilisée est celle de l'échange ionique, plusieurs échanges successifs peuvent être nécessaires pour introduire les quantités requises de métaux.

Quel que soit le mode de réalisation du procédé de préparation du catalyseur selon l'invention, on peut procéder après la calcination dudit catalyseur à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu *ex situ* ou *in situ*, par rapport au lieu d'utilisation dudit catalyseur dans une réaction donnée.

La répartition entre les trois zéolithes, éventuellement entre les quatre zéolithes, de chacun des groupes définis précédemment est telle que la teneur en chacune des zéolithes, présentes dans le catalyseur selon l'invention, peut varier de 1% à 98% poids, de préférence de 2 à 60% poids et de manière encore plus préférée de 4 à 50% poids en pourcentages massiques d'une zéolithe par rapport à l'ensemble des zéolithes introduites dans le catalyseur.

Le catalyseur selon la présente invention est mis en forme sous la forme de grains de différentes formes et dimensions. Il est utilisé en général sous la forme d'extrudés cylindriques ou polylobés tels que bilobés, trilobés, polylobés de forme droite ou torsadée, mais peut éventuellement être fabriqué et employé sous la forme de poudre, pastilles, de tablettes, d'anneaux, de billes, de roues.

Le catalyseur de la présente invention peut éventuellement contenir du soufre. Dans ce cas, le soufre est introduit sur le catalyseur mis en forme, calciné, contenant le ou les métal(ux) cité(s) précédemment, soit *in situ* avant la réaction catalytique, soit *ex situ*. La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction sous hydrogène. Dans le cas d'une sulfuration *in situ*, la réduction, si le catalyseur n'a pas été préalablement réduit, intervient avant la sulfuration. Dans le cas d'une sulfuration *ex situ*, on effectue la réduction puis la sulfuration.

Un autre objet de l'invention est l'utilisation du catalyseur selon l'invention dans des procédés pour la conversion d'hydrocarbures. Plus précisément, la présente invention concerne un procédé d'isomérisation d'une charge comprenant des composés aromatiques à 8 atomes de carbone par molécule réalisé en présence du catalyseur selon l'invention. Ladite charge comprend un mélange de xylènes et de l'éthylbenzène. Ledit procédé est mis en oeuvre en phase gazeuse, préférentiellement en l'absence de toute phase liquide, et en présence d'hydrogène. Ledit procédé est mis en oeuvre généralement selon les conditions opératoires suivantes :

- une température comprise entre 300°C et 500°C, de préférence entre 320°C et 450°C et de manière encore plus préférée entre 340°C et 430°C,
- une pression partielle d'hydrogène comprise entre 0,3 et 1,5 MPa, de préférence entre 0,4 et 1,2 MPa et de manière encore préférée entre 0,7 et 1,2 MPa,
- une pression totale comprise entre 0,45 et 1,9 MPa, de préférence entre 0,6 et 1,5 MPa,
- une vitesse spatiale d'alimentation, exprimée en kilogramme de charge introduite par kilogramme de catalyseur et par heure, comprise entre 0,25 et 30 $h^{-1}$, de préférence entre 1 et 10 $h^{-1}$, et de manière encore plus préférée entre 2 et 6 $h^{-1}$.

Les exemples qui suivent illustrent l'invention sans pour autant en limiter la portée.

**Exemple 1 : Préparation d'un catalyseur à base d'une zéolithe de type structural EUO (comparatif)**

La matière première utilisée est une zéolithe EU-1, brute de synthèse, comprenant le structurant organique, du silicium et de l'aluminium, possédant un rapport atomique Si/Al global égal à 13,6, une teneur pondérale en sodium par rapport au poids en zéolithe EU-1 sèche d'environ 1,5%, correspondant à un rapport atomique Na/Al de 0,6. Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange. A l'issue de ces traitements, la zéolithe EU-1 sous forme $NH_4$ a un rapport Si/Al atomique global égal à 18,3 , une teneur pondérale en sodium par rapport au poids de zéolithe EU-1 sèche de 50 ppm, correspondant à un rapport atomique Na/Al de 0,003. La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 15 % de zéolithe EU-1 sous forme H et 85 % d'alumine.

Le support ainsi obtenu est soumis à un échange anionique avec de l'acide hexachloroplatinique en présence d'un agent compétiteur (acide chlorhydrique), de manière à introduire 0,3% poids de platine par rapport au poids du catalyseur. Le solide humide est ensuite séché à 120°C pendant 12 heures et calciné sous un débit d'air sec à la température de 500°C pendant une heure. Le catalyseur C0 ainsi obtenu contient en poids 15,0% de zéolithe EU-1 sous forme H, 84,7% d'alumine et 0,3% de platine.

**Exemple 2 : préparation de catalyseurs à base d'une zéolithe de type structural EUO, d'une zéolithe 10MR et d'une zéolithe 12MR (conforme à l'invention).**

Les zéolithes utilisées pour préparer les catalyseurs illustrant l'invention sont présentées dans le tableau 1 avec leur composition (rapport atomique Si / Al) et leur teneur résiduelle en sodium. Les zéolithes concernées sont sous forme acide.

La zéolithe EU-1 est synthétisée de la même manière que la procédure donnée à l'exemple 1. Après calcination sous air à 550°C puis après 3 échanges ioniques dans une solution de $NH_4NO_3$ 10N pendant 4h, elle possède un rapport atomique Si/Al de 18,3 et Na/Al de 0,003 correspondant à une teneur en Na de 50 ppm.

Les zéolithes béta, mordénite, Y et ZSM-5 sont des zéolithes commerciales (Zeolyst).

La zéolithe NU-87 a été synthétisée d'après la demande de brevet européen EP-A-0.377.291 ou le brevet EP-B-0.378.916. Elle possède un rapport atomique Si/Al global égal à 17,2, une teneur pondérale en sodium égale à 1256 ppm. Cette zéolithe NU-87 subit, tout d'abord, une calcination dite sèche à 550°C sous flux d'air et d'azote durant 6 heures. Puis, le solide obtenu est soumis à un échange ionique dans une solution de $NH_4NO_3$ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires.

A l'issue de ces traitements, la zéolithe obtenue se trouve sous sa forme H et possède un rapport Si/Al atomique global égal à 33,3, et une teneur en Na de 10 ppm.

La zéolithe IM-5 est synthétisée selon l'exemple n°1 de la demande de brevet EP-A-0.946.416 ou du brevet US 6.136.290, le contenu de chacun de ces documents étant incorporé ici par référence.

Tableau 1 : zéolithes à 10 MR et 12 MR

| zéolithes | Si/Al (FX) | Na (ppm) | Type |
|---|---|---|---|
| Béta | 12,5 | 87 | 12 MR |
| ZSM-5 | 17,5 | 132 | 10 MR |
| Mordénite | 10,0 | 109 | 12 MR |
| IM-5 | 12,0 | 84 | 10 MR |
| NU-87 | 33,3 | 10 | 10MR |
| Y | 15,3 | 63 | 12 MR |
| EU-1 | 18,3 | 50 | |

Les zéolithes, qui se trouvent à l'état de poudre, sont ensuite mélangées mécaniquement puis mises en forme par extrusion avec un gel d'alumine de manière à obtenir, après séchage à 100° C pendant une nuit et calcination à 500°C sous air sec, un support qui contient en poids 15 % de zéolithes (chaque support contenant une zéolithe EU-1, une zéolithe 10 MR et une zéolithe 12 MR) et 85 % d'alumine. La partie zéolithique du support est constituée d'un mélange mécanique de trois zéolithes différentes, réalisé avant la mise en forme. La répartition pondérale des zéolithes dans le support zéolithique ainsi que le type de zéolithes présent dans chaque support sont donnés dans le tableau 2.

Pour la préparation des catalyseurs C2 à C8, le support zéolithique comprenant un mélange de trois zéolithes différentes, à savoir au moins une zéolithe de type structural EUO, au moins une zéolithe 10 MR et au moins une zéolithe 12 MR, est soumis à une imprégnation à sec par une solution de précurseur métallique, soit par une solution d'acide

hexachloroplatinique $H_2PtCl_6$ lorsque le métal est le platine (C2 à C5 et C8) soit par une solution d'acide perrhénique lorsque le métal est le rhénium (C6 et C7). S'agissant du catalyseur C7, le dépôt d'indium est assuré par imprégnation à sec de chlorure d'indium déposé sur le support après l'imprégnation à sec de rhénium et séchage à 120°C pendant une nuit. S'agissant du catalyseur C8, le dépôt étain est assuré par imprégnation à sec du chlorure d'étain déposé sur le support après l'imprégnation à sec de platine et séchage à 120°C pendant une nuit.

Après le dépôt de l'ensemble des métaux sur les différents supports, chaque solide humide est ensuite séché à 120°C pendant 12 heures et calciné sous un débit d'air sec à la température de 500°C pendant une heure. La composition des catalyseurs obtenus est reportée dans le tableau 2.

Tableau 2 : catalyseurs contenant une zéolithe EU-1, une zéolithe 10 MR, une zéolithe 12 MR

| Catalyseur | zéolithe(s) | répartition des zéolithes | % Métal |
|---|---|---|---|
| C1 | EU-1 + ZSM-5 + béta | 50/ 25 /25 | aucun |
| C2 | EU-1 + ZSM-5 + béta | 50/25/25 | 0,3% Pt |
| C3 | EU-1 + ZSM-5 + Mordénite | 50/25/25 | 0,3% Pt |
| C4 | EU-1 + IM-5 + béta | 50/25/25 | 0,3% Pt |
| C5 | EU-1 + Y+ NU-87 | 50/10/40 | 0,3% Pt |
| C6 | EU-1 + ZSM-5 + Mordénite | 50/10/40 | 0,3% Re |
| C7 | EU-1 + ZSM-5 + Mordénite | 50/10/40 | 0,3% Re-0,3%In |
| C8 | EU-1 + ZSM-5 + Mordénite | 50/25/25 | 0,3% Pt-0,3%Sn |

**Exemple 3 : Evaluation des propriétés catalytiques des catalyseurs C0 à C8 en isomérisation d'une coupe C8 aromatique.**

Les performances des catalyseurs C0 à C8 ont été évaluées dans l'isomérisation d'une coupe aromatique comprenant des composés aromatiques à 8 atomes de carbone par molécule, principalement du métaxylène, de l'orthoxylène et de l'éthylbenzène. Les conditions opératoires utilisées sont:

- Température = 390°C,
- pression = 15 bar,
- pression partielle $H_2$ = 12 bar

Les catalyseurs sont préalablement traités avec une charge contenant du dimethyldisulfure (DMDS) en présence d'hydrogène, avec une concentration telle que le rapport atomique soufre sur métal soit de 1,5. Les catalyseurs sont ensuite maintenus pendant 3 heures à 400°C sous débit d'hydrogène puis la charge est injectée.

Les catalyseurs ont été comparés en terme d'activité par la conversion de l'éthylbenzène, et en terme de sélectivité par les pertes nettes à iso-approche à l'équilibre du para-xylène.

La réaction d'isomérisation conduit à des réactions parasites générant trois types de pertes : les pertes vers les paraffines résultant essentiellement des réactions d'ouverture de cycles naphténiques suivies de craquage, les pertes vers les aromatiques formés par les réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes).

Les pertes par craquage (P1) sont des pertes en AC8 sous forme de paraffines (PAR) ayant de un à huit atomes de carbone :

$$P1(\%poids) = 100 \times [(\%PAR_{effluent} \times poids\ d'effluent) - (\%PAR_{charge} \times poids\ de\ charge)] / (\%AC8_{charge} \times poids\ de\ charge)$$

Les pertes par dismutation/transalkylation (P2) sont des pertes en AC8 sous forme de naphtènes autres que N8, de toluène, de benzène et de C9+ aromatiques (OAN) :

$$P2(\%poids) = 100 \times [(\%OAN_{effluent} \times poids\ d'effluent) - (\%OAN_{charge} \times poids\ de\ charge)] / (\%AC8_{charge} \times poids\ de\ charge)$$

La somme des pertes P1 et P2 représente les pertes nettes.

Les données présentées dans le tableau 3 ont été obtenues à iso-conditions expérimentales et iso-approche à l'équilibre (98%).

Tableau 3 : évaluation catalytique des catalyseurs C0 à C8

| Catalyseur | C0 | C1 | C2 | C3 | C4 | C5 | C6 | C7 | C8 |
|---|---|---|---|---|---|---|---|---|---|
| conversion EB (%) | 61,3 | 61,9 | 62,8 | 63,2 | 62,2 | 63,1 | 64,2 | 64,3 | 63,4 |
| pertes nettes (% poids) | 6,2 | 5,9 | 5,7 | 5,1 | 4,8 | 4,9 | 5,8 | 5,4 | 4,9 |

Les catalyseurs C1 à C8 selon l'invention qui comprennent chacun une zéolithe de type structural EUO, une zéolithe 10 MR et une zéolithe 12 MR, conduisent à une meilleure conversion de l'éthylbenzène et à une diminution des pertes nettes traduisant des réactions secondaires limitées par rapport aux performances obtenues au moyen d'un catalyseur C0 uniquement à base d'une zéolithe de type structural EUO. L'introduction d'un métal, le plaine ou le rhénium, sur le support (C2 à C6) permet également une diminution des pertes nettes et une meilleure conversion de l'éthylbenzène par rapport à un catalyseur C1 dépourvu de métal. L'introduction d'un métal additionnel, de l'étain ou de l'indium (C8 respectivement C7), permet une diminution des pertes nettes encore plus significative et une conversion de l'éthylbenzène encore meilleure par rapport aux performances obtenues avec un catalyseur ne contenant qu'un seul métal (C3 respectivement C6).

**Revendications**

1.  Catalyseur comportant au moins une zéolithe de type structural EUO, au moins une zéolithe présentant des canaux dont l'ouverture est définie par un anneau à 10 atomes d'oxygène (10 MR), au moins une zéolithe présentant des canaux dont l'ouverture est définie par un anneau à 12 atomes d'oxygène (12 MR) et ayant un type structural différent de EUO et au moins une matrice minérale poreuse.

2.  Catalyseur selon la revendication 1 comportant au moins un métal choisi parmi les métaux des groupes VIB, VIIB et VIII de la classification périodique des éléments.

3.  Catalyseur selon la revendication 2 dans lequel ledit métal est choisi parmi les métaux du groupe VIII.

4.  Catalyseur selon la revendication 3 dans lequel ledit métal choisi parmi les métaux du groupe VIII est le platine.

5.  Catalyseur selon l'une des revendications 1 à 4 dans lequel la zéolithe présentant des canaux dont l'ouverture est définie par un anneau à 10 atomes d'oxygène est choisie parmi les zéolithes ZSM-5, NU-87, IM-5, ferrierite, NU-85 et ZSM-22.

6.  Catalyseur selon la revendication 5 dans lequel la zéolithe présentant des canaux dont l'ouverture est définie par un anneau à 10 atomes d'oxygène est une zéolithe ZSM-5.

7.  Catalyseur selon l'une des revendications 1 à 6 dans lequel la zéolithe présentant des canaux dont l'ouverture est définie par un anneau à 12 atomes d'oxygène est choisie parmi les zéolithes beta, Y, mordénite, ZSM-12, la mazzite et la boggsite.

8.  Catalyseur selon la revendication 7 dans lequel la zéolithe présentant des canaux dont l'ouverture est définie par un anneau à 12 atomes d'oxygène est la mordénite.

9.  Catalyseur selon la revendication 7 dans lequel la zéolithe présentant des canaux dont l'ouverture est définie par un anneau à 12 atomes d'oxygène est la béta.

**10.** Catalyseur selon l'une des revendications 1 à 9 dans lequel ladite zéolithe de type structural EUO est une zéolithe EU-1.

**11.** Catalyseur selon l'une des revendications 1 à 10 comportant au moins une zéolithe présentant des canaux dont l'ouverture est définie par un anneau à 8 atomes d'oxygène (8 MR).

**12.** Catalyseur selon l'une des revendications 2 à 11 comportant au moins un métal choisi parmi les métaux des groupes IIIA et IVA.

**13.** Catalyseur selon l'une des revendications 1 à 12 comportant du soufre.

**14.** Procédé d'isomérisation d'une charge comprenant des composés aromatiques à 8 atomes de carbone par molécule réalisé en présence d'un catalyseur selon l'une des revendications 1 à 13.

**15.** Procédé d'isomérisation selon la revendication 14 tel que ladite charge comprend un mélange de xylènes et de l'éthylbenzène.

**16.** Procédé d'isomérisation selon la revendication 14 ou la revendication 15 tel qu'il est réalisé à une température comprise entre 300 et 500°C, avec une pression partielle d'hydrogène comprise entre 0,3 et 1,5 MPa, avec une pression totale comprise entre 0.45 et 1,9 MPa et une vitesse spatiale d'alimentation, exprimée en kilogramme de charge introduite par kilogramme de catalyseur et par heure, comprise entre 0.25 et 30 h$^{-1}$.

**Claims**

**1.** A catalyst comprising at least one zeolite with structure type EUO, at least one zeolite having channels the opening to which is defined by a ring of 10 oxygen atoms (10 MR), at least one zeolite having channels the opening to which is defined by a ring of 12 oxygen atoms (12 MR) and having a structure type different from EUO, and at least one porous mineral matrix.

**2.** A catalyst according to claim 1, comprising at least one metal selected from metals from groups VIB, VIIB and VIII of the periodic table of the elements.

**3.** A catalyst according to claim 2, in which said metal is selected from metals from group VIII.

**4.** A catalyst according to claim 3, in which said metal which is selected from metals from group VIII is platinum.

**5.** A catalyst according to one of claims 1 to 4, in which the zeolite with channels the opening to which is defined by a ring of 10 oxygen atoms is selected from ZSM-5, NU-87, IM-5, ferrierite, NU-85 and ZSM-22.

**6.** A catalyst according to claim 5, in which the zeolite with channels the opening to which is defined by a ring of 10 oxygen atoms is a ZSM-5 zeolite.

**7.** A catalyst according to one of claims 1 to 6, in which the zeolite with channels the opening to which is defined by a ring of 12 oxygen atoms is selected from beta, Y, mordenite, ZSM-12, mazzite and boggsite zeolites.

**8.** A catalyst according to claim 7, in which the zeolite with channels the opening to which is defined by a ring of 12 oxygen atoms is mordenite.

**9.** A catalyst according to claim 7, in which the zeolite with channels the opening to which is defined by a ring of 12 oxygen atoms is beta zeolite.

**10.** A catalyst according to one of claims 1 to 9, in which said zeolite with structure type EUO is an EU-1 zeolite.

**11.** A catalyst according to one of claims 1 to 10, comprising at least one zeolite with channels the opening to which is defined by a ring of 8 oxygen atoms (8 MR).

**12.** A catalyst according to one of claims 2 to 11, comprising at least one metal selected from metals from groups IIIA and IVA.

13. A catalyst according to one of claims 1 to 12, comprising sulphur.

14. A process for isomerizing a feed comprising aromatic compounds containing eight carbon atoms per molecule, carried out in the presence of a catalyst according to one of claims 1 to 13.

15. An isomerization process according to claim 14, in which said feed comprises a mixture of xylenes and ethylbenzene.

16. An isomerization process according to claim 14 or claim 15, carried out at a temperature in the range 300°C to 500°C, with a partial pressure of hydrogen in the range 0.3 to 1.5 MPa, with a total pressure in the range 0.45 to 1.9 MPa and an hourly space velocity, expressed in kilograms of feed introduced per kilogram of catalyst per hour, in the range 0.25 to 30 $h^{-1}$.

**Patentansprüche**

1. Katalysator, der mindestens einen Zeolithen vom Strukturtyp EUO, mindestens einen Zeolithen, der Kanäle aufweist, deren Öffnung durch einen Ring mit 10 Sauerstoffatomem definiert ist (10 MR), mindestens einen Zeolithen, der Kanäle aufweist, deren Öffnung durch einen Ring mit 12 Sauerstoffatomen definiert ist (12 MR), und einen von EUO verschiedenen Strukturtyp hat, und mindestens eine poröse Mineralmatrix umfasst.

2. Katalysator nach Anspruch 1, der mindestens ein Metall umfasst, ausgewählt aus den Metallen der Gruppen VIB, VIIB und VIII des Periodensystems der Elemente.

3. Katalysator nach Anspruch 2, wobei das Metall ausgewählt ist aus den Metallen der Gruppe VIII.

4. Katalysator nach Anspruch 3, wobei das Metall, das aus den Metallen der Gruppe VIII ausgewählt ist, Platin ist.

5. Katalysator nach einem der Ansprüche 1 bis 4, wobei der Zeolith, der Kanäle aufweist, deren Öffnung durch einen Ring aus 10 Sauerstoffatomen definiert ist, ausgewählt ist aus den Zeolithen ZSM-5, NU-87, IM-5, Ferrierit, NU-85 und ZSM-22.

6. Katalysator nach Anspruch 5, wobei der Zeolith, der Kanäle aufweist, deren Öffnung durch einen Ring mit 10 Sauerstoffatomem definiert ist, ein ZSM-5-Zeolith ist.

7. Katalysator nach einem der Ansprüche 1 bis 6, wobei der Zeolith, der Kanäle aufweist, deren Öffnung durch einen Ring aus 12 Sauerstoffatomen definiert ist, ausgewählt ist aus den Zeolithen Beta, Y, Mordenit, ZSM-12, Mazzit und Boggsit.

8. Katalysator nach Anspruch 7, wobei der Zeolith, der Kanäle aufweist, deren Öffnung durch einen Ring mit 12 Sauerstoffatomem definiert ist, Mordenit ist.

9. Katalysator nach Anspruch 7, wobei der Zeolith, der Kanäle aufweist, deren Öffnung durch einen Ring mit 12 Sauerstoffatomem definiert ist, der Beta-Zeolith ist.

10. Katalysator nach einem der Ansprüche 1 bis 9, wobei der Zeolith des Strukturtyps EUO ein EU-1-Zeolith ist.

11. Katalysator nach einem der Ansprüche 1 bis 10, der mindestens einen Zeolithen umfasst, der Kanäle aufweist, deren Öffnung durch einen Ring mit 8 Sauerstoffatomem definiert ist (8 MR).

12. Katalysator nach einem der Ansprüche 2 bis 11, der mindestens ein Metall ausgewählt aus den Metallen der Gruppen IIIA und IVA umfasst.

13. Katalysator nach einem der Ansprüche 1 bis 12, der Schwefel umfasst.

14. Verfahren zur Isomerisierung einer Beschickung, die aromatische Verbindungen mit 8 Kohlenstoffatomen je Molekül umfasst, das in Gegenwart eines Katalysators nach einem der Ansprüche 1 bis 13 ausgeführt wird.

**15.** Verfahren zur Isomerisierung nach Anspruch 14, so dass die Beschickung eine Mischung aus Xylenen und Ethylbenzen umfasst.

**16.** Verfahren zur Isomerisierung nach Anspruch 14 oder Anspruch 15, so dass es bei einer Temperatur im Bereich zwischen 300 und 500 °C, mit einem partiellen Wasserstoffdruck im Bereich zwischen 0,3 und 1,5 MPa, mit einem Gesamtdruck im Bereich zwischen 0,45 und 1,9 MPa und einer Zufuhrraumgeschwindigkeit, ausgedrückt in Kilogramm eingeführter Beschickung pro Kilogramm Katalysator und pro Stunde, im Bereich zwischen 0,25 und 30 $h^{-1}$ ausgeführt wird.

## RÉFÉRENCES CITÉES DANS LA DESCRIPTION

**Documents brevets cités dans la description**

- EP 0923987 A1
- EP 42226 B1
- US 4640829 A
- EP 51318 A1
- US 5446234 A
- EP 0946416 A
- US 6136290 A
- EP 0377291 A
- EP 0378916 B

**Littérature non-brevet citée dans la description**

- **W.M. Meier ; D.H. Olson ; Ch. Baerlocher.** Atlas of Zeolite Structure Types. 2001
- *Zeolites,* 1988, vol. 8, 74
- **W. M Meier ; D. H. Olson ; Ch. Baerlocher.** Atlas of Zeolite Framework Types. Elsevier, 2001